# EUROPEAN PATENT SPECIFICATION

(11) **EP 3 233 135 B1**
(45) Date of publication and mention of the grant of the patent: **10.06.2020**
(21) Application number: 15825836.8
(22) Date of filing: 14.12.2015
(51) Int. Cl.: A61K 49/18, A61K 49/14, B82Y 15/00

(54) **ALBUMIN NANOPARTICLES ENCAPSULATING GADOLINIUM AND METHOD OF SYNTHESIS THEREOF**
ALBUMINNANOPARTIKEL UMHÜLLEND GADOLINIUM UND VERFAHREN ZUR SYNTHESE DAVON
NANOPARTICULES D'ALBUMINE ENCAPSULANT DU GADOLINIUM ET LEUR PROCÉDÉ DE SYNTHÈSE

(30) Priority: 16.12.2014 IT TO20141048
(43) Date of publication of application: 25.10.2017
(73) Proprietor: Fondazione Istituto Italiano di Tecnologia, 16163 Genova (IT)
(72) Inventor: SABELLA, Stefania, 73100 Lecce (IT); MALVINDI, Maria Ada, 73100 Lecce (IT); TORINO, Enza, 80132 Napoli (IT); POMPA, Pier Paolo, 73100 Lecce (IT); CINGOLANI, Roberto, 16014 Ceranesi (Genova) (IT); NETTI, Paolo, 80122 Napoli (IT)
(74) Representative: Comoglio, Elena
(86) International application number: PCT/IB2015/059578
(87) International publication number: WO 2016/097963

(56) References cited:
- WO-A1-2012/113733
- US-A- 5 336 762
- LI ET AL: "Preparation and characterization of sodium ferulate entrapped bovine serum albumin nanoparticles for liver targeting", INTERNATIONAL JOURNAL OF PHARMACEUTICS, ELSEVIER BV, NL, vol. 349, no. 1-2, 27 December 2007 (2007-12-27), pages 274-282, XP022402493, ISSN: 0378-5173, DOI: 10.1016/J.IJPHARM.2007.08.001
- BANSAL A ET AL: "Design and development of paclitaxel-loaded bovine serum albumin nanoparticles for brain targeting", ACTA PHARMACEUTICA 20110601 CROATIAN PHARMACEUTICAL SOCIETY HRV, vol. 61, no. 2, 1 June 2011 (2011-06-01), pages 141-156, XP002743646, ISSN: 1330-0075
- SADEGHI R ET AL: "The effect of different desolvating agents on BSA nanoparticle properties and encapsulation of curcumin", JOURNAL OF NANOPARTICLE RESEARCH, KLUWER ACADEMIC PUBLISHERS, DORDRECHT, NL, vol. 16, no. 9, 29 August 2014 (2014-08-29), pages 1-14, XP035398141, ISSN: 1388-0764, DOI: 10.1007/S11051-014-2565-1 [retrieved on 2014-08-29]
- WATCHARIN WARALEE ET AL: "Biodegradable human serum albumin nanoparticles as contrast agents for the detection of hepatocellular carcinoma by magnetic resonance imaging", EUROPEAN JOURNAL OF PHARMACEUTICS AND BIOPHARMACEUTICS, vol. 87, no. 1, May 2014 (2014-05), pages 132-141, XP029028386, ISSN: 0939-6411, DOI: 10.1016/J.EJPB.2013.12.010 cited in the application

## Description

The present invention relates to albumin nanoparticles encapsulating a chemical species containing Gd³⁺, as well as the method of synthesis of the said nanoparticles.

Gadolinium is a metal belonging to the lanthanide class. It is widely used in the electronics industry (e.g., for the production of computer memories), in the metalworking industry and in medical diagnostics.

Gadolinium, in the ionic form (Gd³⁺), represents the only metal not belonging to the group of transition metals to show magnetic properties at room temperature. It has 7 unpaired electrons and a strong relaxivity signal (hydrogen-proton spin lattice relaxation effect) in comparison with other paramagnetic ions. It is therefore used as a contrast agent for magnetic resonance in radiology. However, gadolinium is a very unstable and toxic molecule, so its delivery *in vivo* is not simple.

The chelated forms of gadolinium allow to reduce toxic effects *in vivo,* even if they do not abolish its toxicity completely. Several chemical strategies of complexing are described in the state of the art for the attainment of stable gadolinium complexes with different molecules, such as for example ethylene diamine tetraacetic acid (EDTA) and diethylene diamine pentaacetic acid or pentetic acid (GTPA). Pentetic acid, also known as gadopentic acid, is among the most used molecules, since it forms a very stable complex with gadolinium and is therefore well tolerated by humans. Gadopentic acid is generally administered as a dimeglumine salt, wherein the protonated form of the amino sugar dimeglumine forms a stable salt with the ionic form of gadopentic acid. Administered intravenously, gadopentate dimeglumine was among the first contrast agents to be approved by the FDA for marketing. A few examples of commercially available contrast agents based on gadolinium complexes are listed in Table 1 below. It is important to stress that all the listed compounds cause nephrogenic systemic fibrosis (NSF).

**Table 1**

| **Trade Name** ® | **Chemical Name** | **Company** |
|---|---|---|
| Magnevist | Gadopentetate dimeglumine (Gd-DTPA) | Bayer Schering Pharma |
| Dotarem | gadoterate (Gd-GBCA) | Guerbet |
| MultiHance | gadobenate dimeglumine (Gd-BOPTA) | Bracco |
| ProHance | gadoteridol | Bracco |
| Omniscan | gadodiamide | GE Healthcare |
| OptiMARK | gadoversetamide | Mallinckrodt |

Another known class of diagnostic drugs that employ gadolinium consists of gadolinium complexes that bind the protein albumin, wherein the bond with albumin is a covalent or an electrostatic bond (Huang, et al. "Gd-based macromolecules and nanoparticles as magnetic resonance contrast agents for molecular imaging" Curr Top Med Chem. 2013, 13(4): 411-421).

Ogan, M.D., et al. "Albumin labeled with Gd-DTPA. An intravascular contrast-enhancing agent for magnetic resonance blood pool imaging: preparation and characterization", Investigative radiology 22, 665-671 (1987), describes a method for producing a Gd-DTPA molecule bound to albumin. The molecule is used as an intravascular MRI contrast agent, wherein an albumin molecule is covalently linked to approximately 19 molecules of gadolinium-DTPA. Gd-DTPA bound to albumin, having a higher molecular weight, has a greater selectivity for the vessels, as well as a longer half-life.

The electrostatically bonded complexes, designated as "gadolinium-based blood pool agents", show properties of reversible binding to the protein. This property reduces the relaxation time, thereby causing an increase in the relaxivity and the sensitivity of the MRI signal (P. Caravan, "Protein-targeted gadolinium-based magnetic resonance imaging (MRI) contrast agents: design and mechanism of action", Acc. Chem. Res. 42 7 (2009) 851-862). However, the above compounds have some disadvantages. The quick removal of gadolinium, and therefore its rapid *in vivo* clearance, (as determined by typical low values of the affinity constants) leads, in some cases, to poor-quality MRI images.

In summary, the drawbacks of the gadolinium and albumin complexes described in the state of the art, in which there is a covalent or an electrostatic bond, are the following:
For covalent complexes, the toxicity of the molecule incorporating gadolinium and the MRI signals that sometimes are not strong enough.

For electrostatic complexes (gadolinium-based blood pool agents), the instability of the gadolinium-protein complex may affect the quality of the MRI image; the complex does not remain stable for a period of time long enough for the acquisition of the MRI signal.

In both types of complexes, moreover, gadolinium can be subject to rapid degradation *in vivo.* Finally, gadolinium is highly toxic because it is not encapsulated. The protein molecules also have a low ability to bind the ion.

Several methodologies for the synthesis of albumin nanoparticles incorporating chemical species containing gadolinium are described in the state of the art.

Stollenwerk et al. "Albumin-based nanoparticles as magnetic resonance contrast agents: I. Concept, first syntheses and characterization", Histochem Cell Biol (2010) 133:3 describe albumin-polylactic acid nanoparticles loaded with gadolinium and having sizes between 20 and 40 nm. The nanoparticles are obtained by emulsification. The Gd³⁺ ions are in the form of DTPA-Gd chelates. Gd-DTPA is covalently linked to albumin before the process for the formation of the nanoparticles, which severely limits the load capacity of the nanoparticles.

Watcharin et al. Eur J Pharm Biopharm. 2014 May; 87 (1) :132-41. doi: 10.1016 /j.ejpb.2013.12.010 . Epub 2013 Dec 21, describe a method for the manufacture of nanoparticles of human serum albumin (HSA) which are covalently linked to gadoliniumdiethylene triamine pentaacetic acid (Gd-DTPA) by using carbodiimide. The nanoparticles have a diameter of about 200 nm.

WO2012/113733 describes nanoparticles of HSA which are covalently linked to Gd-DTPA, wherein the nanoparticles have a diameter of 100-300 nm.

In order to overcome these and other drawbacks of the prior art, the present inventors provided cross-linked albumin nanoparticles having a size of between 50 nm and 80 nm, and encapsulating a plurality of Gd³⁺ -bearing molecules without covalent bonding.

In a preferred embodiment of the nanoparticles of the invention, the chemical species containing Gd³⁺ is a Gd³⁺ salt or a Gd³⁺ complex, preferably GdCl₃ or Gd-DTPA.

In yet another preferred embodiment, albumin is cross-linked with glutaraldehyde.

Advantageously, in the nanoparticles of the invention Gd³⁺ is encapsulated in a stable way and is not released in the medium under conditions similar to the physiological ones. This causes the nanoparticles to have a protective action against the inherent toxicity of gadolinium, preserving, at the same time, its paramagnetic characteristics. In addition, encapsulation of gadolinium in the nanostructure leads to an increase of the MRI signal with a considerable reduction of the T1 signal and an increase of relaxivity (r1).

Moreover, the nanoparticles of the invention have the ability to incorporate multiple gadolinium molecules (for example, gadolinium chloride, Gd-DTPA, etc.) through the use of a single synthesis process and without resorting to any type of chelation or covalent or electrostatic complexing. This causes, as a result, a high load capacity of the gadolinium molecules. The inventors have estimated, in fact, that a nanoparticle of the invention of about 50 nm contains approximately 5000 gadolinium molecules, when the synthesis takes place in molar concentration ratios of 1:1. These values increase considerably with the increase in the size of the nanoparticles or with the increase in molar concentration ratios between the protein and gadolinium (for example 1:2 between the protein and gadolinium), with the possibility of obtaining a fine control over the final concentration of encapsulated ionic molecules.

The nanoparticles of the invention also have useful tumoritropic properties (Matsumura, Y. & Maeda, H. A New Concept for Macromolecular Therapeutics in Cancer-Chemotherapy - Mechanism of Tumoritropic Accumulation of Proteins and the Antitumor Agent Smancs. Cancer Res 46, 6387-6392 (1986);), as well as other intrinsic properties (typical of nanovectors) which determine the possibility of an EPR effect, active or passive targeting, controlled release.

Even the controlled size of the nanoparticles of the invention is an important feature, which affects their *in vivo* behaviour after intravenous injection. With the methods of the prior art for the synthesis of BSA nanoparticles containing MRI contrast agents, it has not been possible to obtain a satisfactory control of this parameter. Sub-150-nm particles may in fact have the advantage of a higher extravasation and therefore of reaching the tumour mass more easily through passive (for example, through the discontinuous holes of the tumour mass) or active (by the use of signal molecules) targeting processes.

In addition, the nano-dimensions of the particles of the invention combine with the innate tumoritropic properties (i.e., specific recognition of albumin by receptors present on the tumour cells) of the BSA molecules, leading to an improvement in the ability to penetrate the tumour sites. Finally, thanks to the confinement of Gd³⁺ in nanocavities, nanostructuring allows to increase the relaxivity and improve the MRI signal >Ananta, J. S., et al. Geometrical confinement of gadolinium-based contrast agents in nanoporous particles enhances T1 contrast. Nat Nanotechnol 5, 815-821 (2010)). Encapsulation of gadolinium also prevents a rapid clearance of the ions *in vivo* (sustained effect and better MRI imaging resolution) and, at the same time, it reduces the intrinsic toxicity of Gd³⁺.

A second aspect of the invention is the method of synthesis of the nanoparticles of the invention.

This method is based on the coacervation process. Coacervation is a desolvation process wherein aqueous solutions of albumin are slowly desolvated by dropwise addition of a desolvating agent (typically acetone or ethanol), under constant stirring, temperature and pH conditions. Desolvation triggers a phase separation of the albumin molecules, leading to the formation of self-assembled albumin nanoparticles. After their formation, the albumin nanoparticles are not stable enough, so they need to be stabilized by cross-linking with the use of glutaraldehyde or other cross-linking polymeric agents. The subsequent purification of the nanoparticles is typically achieved by centrifugation (Sailaja A.K., Amareshwar P., Preparation of BSA nanoparticles by desolvation technique using acetone as desolvating agent, International Journal of Pharmaceutical Sciences and Nanotechnology, Volume 5, Issue 1, 2012.).

The present inventors have developed a method for the synthesis of albumin nanoparticles, based on the coacervation process, which allows to obtain a fine control over the size of the nanoparticles and the dispersion of the nanoparticles in solution.

Therefore, a method of synthesis of cross-linked albumin nanoparticles encapsulating a Gd³⁺ -bearing chemical species falls within the scope of the present invention, comprising the steps of:
- desolvating an aqueous solution comprising albumin and a Gd³⁺ -bearing chemical species by the addition to said aqueous solution of a desolvating agent, in order to obtain albumin nanoparticles encapsulating a plurality of molecules of the Gd³⁺ -bearing chemical species; and
- stabilizing the nanoparticles obtained in the previous step by cross-linking of albumin by using a cross-linking agent,
characterized in that the concentration of albumin in the desolvation reaction volume is comprised between 0.1 and 0.5% (w/v), that the molar concentration ratio between albumin and the Gd³⁺ -bearing chemical species in the desolvation reaction volume is comprised between 1:0.5 and 1:2, and that the desolvating agent is acetone.

In a preferred embodiment, the volume ratio between acetone and water in the desolvation step is preferably comprised between 1.25 and 4.

In another preferred embodiment, the cross-linking of albumin is achieved by using a glutaraldehyde aqueous solution at a concentration of between 2 and 8% (v/v).

In yet another preferred embodiment, the method comprises a further step of purification of the nanoparticles by centrifugation.

The use of the above-indicated reaction conditions advantageously and surprisingly allows for the achievement of nanoparticles with a controlled size in the range of 50-80 nm, as well as a fine control of their dispersion in solution. The following Table 2 shows the size of the obtained particles under various reaction conditions, within the ranges characterizing the method of the invention.

**Table 2**

| Size (nm) | %BSA (g/100 ml)* | BSA/Gd³⁺ ratio** | Desolv. agent/water*** | GTA(%) | Time (min) of centrifugation at 16,000 g, and 20 °C |
|---|---|---|---|---|---|
| | | | a: acetone | | |
| | | | b: ethanol | | |
| 50 | 0.1 | 1:0.5; 1:1; 1:2 | ^{a} 4 | 8 | 90 |
| 70 | 0.5 | 1:0.5; 1:1; 1:2 | ^{a} 4 | 8 | 60 |
| 80 | 0.5 | 1:0.5; 1:1; 1:2 | ^{a} 5 | 8 | 60 |
| 90 | 0.1 | 1:0.5; 1:1; 1:2 | ^{b} 4 | 8 | 60 |
| 100 | 0.5 | 1:0.5; 1:1; 1:2 | ^{b} 1.25 | 8 | 60 |
| 150 | 0.5 | 1:0.5; 1:1; 1:2 | ^{b} 2.5 | 8 | 40 |

| | | | | | |
|---|---|---|---|---|---|
| * : the final volume of the desolvation reaction is 10 mL. The concentration values relate to the final desolvation reaction volume ** the ratio is calculated as the molar concentration *** the ratio is calculated by volume | | | | | |

For the purification, the 50-nm nanoparticles were centrifuged for 90 minutes at 16,000 g at T= 20 °C; the 70-100-nm ones were centrifuged for 60 min at 16,000 g at T= 20 °C; the 150-nm ones were centrifuged for 40 minutes at 16,000 g at T= 20 °C.

The comparison between the first and the fourth row in Table 2 shows that in like-for-like reaction conditions, the use of acetone instead of ethanol as the desolvating agent leads to a net decrease in the size of the nanoparticles, from 90 nm (ethanol) to 50 nm (acetone). The amount of GTA used in this synthesis process ranges between 2-8%. It is important to emphasize that nanoparticles produced with 2% GTA and containing gadolinium have the same chemical-physical stability as the nanoparticles produced with 8% GTA. This is important considering that GTA is an inherently toxic agent. Furthermore, nanoparticles obtained with 2% GTA produce the same T1, and thus relaxivity r1, signal as nanoparticles produced with 8% GTA.

The experimental section that follows is provided for illustration purposes.

### Example 1: synthesis of nanoparticles according to the invention

0.01 g of powdered albumin (BSA) is dissolved in 2 mL of Milli-Q water (75.76 µM) and 15 µL of gadolinium chloride (GdCl3) is added from a 0.01 M stock solution in order to have a molar concentration ratio of 1:1 between the two components. The solution thus obtained was subjected to constant stirring (400 rpm), desolvation was achieved by dropwise addition of 8.0 ml of acetone, at a defined speed of 1 mL/min with a syringe pump. An opalescent solution was obtained spontaneously at room temperature. After the desolvation process, glutaraldehyde (GTA) 8% in water (300 µL) was added to obtain cross-linking. The mixture was stirred for 24 hours. After the reaction was completed, the nanoparticles were recovered by centrifugation (16,000 x g, 40 min, 20 °C), the supernatant was discarded, and the nanoparticles were dispersed in water. This purification step was repeated 5 times. Ultrasound treatment for 5 minutes was used to fully disperse the precipitate in the solvent. The above-mentioned reaction conditions led to the obtainment of 50 nm-sized albumin nanoparticles containing gadolinium salt in 1:1 ratios.

To produce BSA nanoparticles of the other sizes shown in Table 2 containing another Gd³⁺ -bearing chemical species (a salt or a complex of Gd³⁺) they were mixed in concentration ratios of 1:0.5, 1:1, or 1:2. The resulting solutions were left to desolvate with controlled amounts of acetone/water or ethanol/water to form BSA-Gd NPs according to the method described above and the parameters indicated in Table 2.

### Example 2: characterisation of nanoparticles according to the invention

The physical-chemical characterization of the gadolinium-BSA nanoparticles according to the invention was performed by dynamic light scattering (DLS), measurement of the zeta potential, TEM characterization, determination of Gd³⁺ encapsulation in the BSA nanoparticles, and MRI measurements.

### 2.1 Dynamic light scattering and measurement of the zeta potential

The size distribution and surface charge of the BSA nanoparticles and the gadoliniumdoped BSA nanoparticles obtained according to the method described in example 1 in combination with Table 2, were measured in solution via dynamic light scattering (Zetasizer Nano ZS90, Malvern, USA). Measurements were made at 25 °C in aqueous solutions (pH = 7). The DLS spectra obtained are not shown in the figures.

### 2.2 TEM characterization

The morphology of the nanoparticles was assessed with a transmission electron microscope (JEOL JEM 1011) at an accelerating voltage of 100 kV. The TEM specimens were prepared by pouring a diluted solution of nanoparticles in water on copper grids coated with carbon (Formvar/Carbon 300 Mesh Cu). The TEM images are not shown in the figures.

### 2.3 Assessment of Gd³⁺ encapsulation in the BSA nanoparticles

The efficiency of gadolinium encapsulation in the BSA nanoparticles was evaluated by measuring the amount of gadolinium in the BSA nanoparticles through elemental analysis by ICP-AES. The elemental analysis was carried out by inductively coupled plasma - atomic emission spectroscopy (ICP-AES) with an Agilent 720/730 spectrometer. The nanoparticle samples were centrifuged, and the obtained pellets and supernatants were digested with nitric acid using a mineralizer, diluted to 5 mL with ultrapure water, and finally the resulting solutions were analysed by ICP-AES.

### 2.4 MRI Measurements

The gadolinium-BSA nanoparticles (GdCl₃, Gd-DTPA) in different concentrations and size were analysed at two different magnetic fields (1.5 T or 3T MR) (Philips Achieva 1.5T and 3T). Gd-DTPA aqueous solutions, at corresponding concentrations, were used as reference material.

For T1 measurements, the inversion recovery sequence was used with the following parameters: TR = 2500 ms, TE = 12 ms; TI = 50, 100, 200, 400, 800, 1100, 1800 ms; FOV= 180x146 mm; the smallest slice selected for the acquisition is equal to 4 mm and the images of the modules were acquired and analysed by the MRMap mapping software. After the analysis, T1 maps were generated and the images were later scored using the corresponding TI values. The relaxivity (r1), i.e. the ability of paramagnetic material to act as an MRI contrast agent, is described as the variation of the relaxation rate 1/Ti (s-1) of water protons per mM concentration of the contrast agent and can be calculated using the expression ri = (1/Ti-1/Tid) / [CA], in which Ti is the relaxation time in the presence of the contrast agent, and [CA] is the concentration of the paramagnetic contrast agent (mM). The relaxivity (r1) and the associated uncertainty were calculated using a weighted linear regression (R1 = 1/T1 s-1 relaxation rate plotted against the mM concentration of gadolinium and r1 mM-1 s-1 is the slope of the curve fitting the experimental points). All experiments were performed in triplicate.

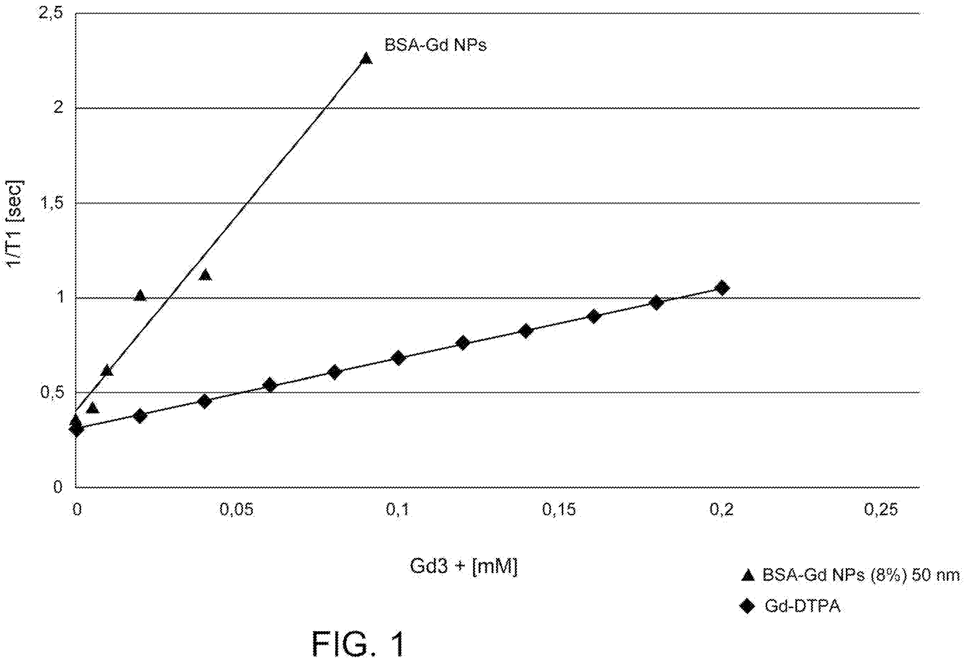

The increase in the contrast of the 50-nm BSA-gadolinium nanoparticles was examined using a clinical MRI scanner at 1.5T and 3T. The signal generated by the nanoparticles was compared with that produced by aqueous solutions of Gd-DTPA, which is the reference material. When equal ion concentrations were compared, a significant increase in the r1 signal was observed, due to the encapsulation of gadolinium in the nanostructure (Figure 1).

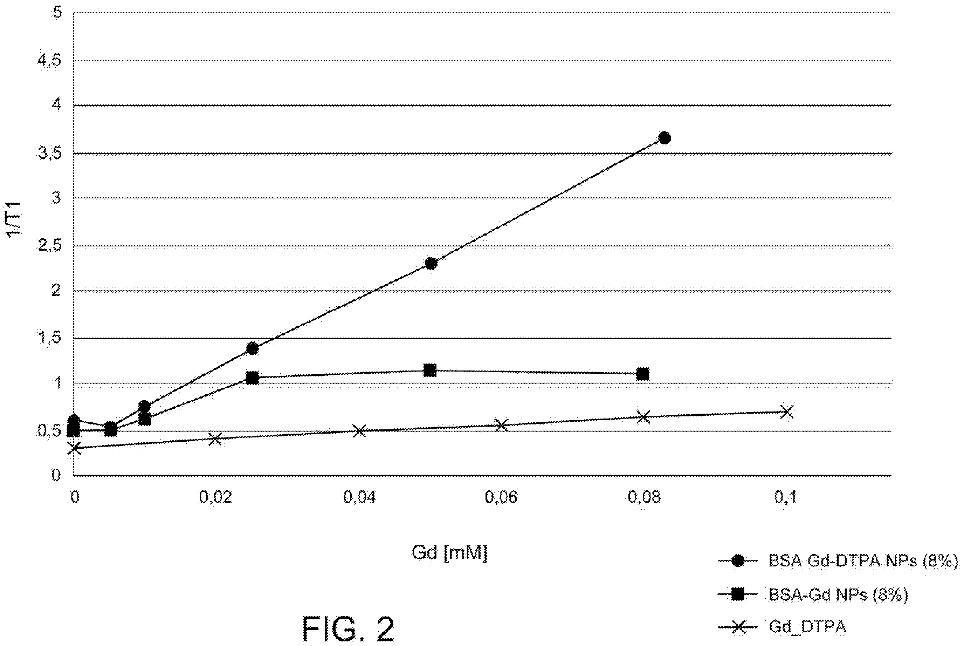

Figure 2 shows the increased contrast of 150-nm BSA nanoparticles incorporating gadolinium ions or Gd-DTPA in which the gadolinium ions are chelated to DTPA molecules. The signals are compared with those obtained from the commercial reference (Gd-DTPA). In both cases, an increase in the contrast can be observed, however, this signal is significantly lower in the case of the nanoparticles incorporating Gd-DTPA. Similar results were obtained with smaller sizes.

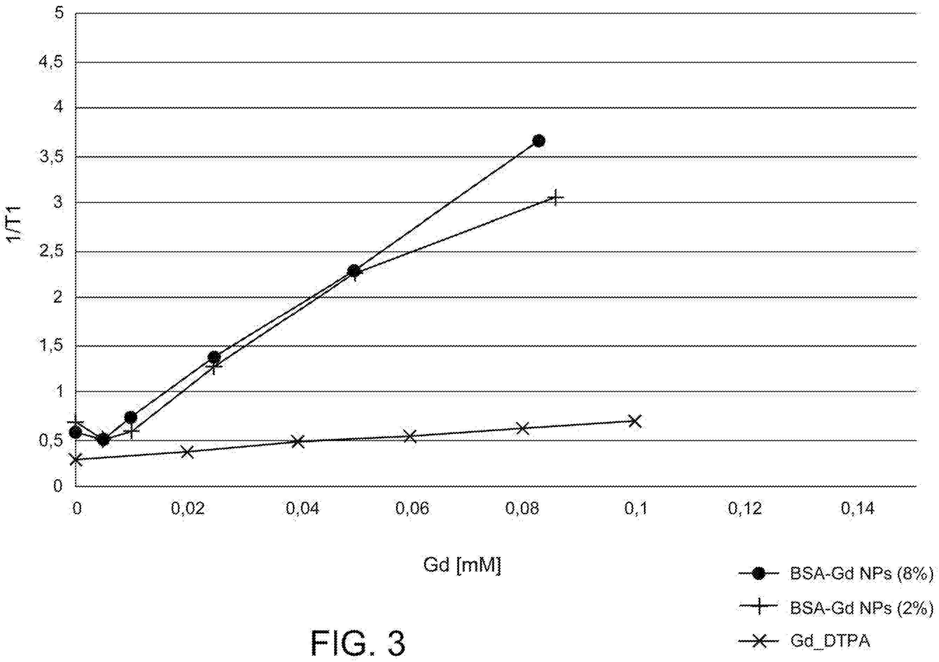

Figure 3 shows the increased contrast of BSA-gadolinium nanoparticles of approximately 150 nm produced with two different concentrations of GTA (8% and 2%). The results obtained were comparable for both types of nanoparticles.

## Claims

1. A cross-linked albumin nanoparticle loaded with a plurality of molecules of a Gd³⁺-bearing chemical species, **characterized in that** the size of the nanoparticle is comprised between 50 nm and 80 nm and that the molecules of the Gd³⁺ -bearing chemical species are encapsulated by albumin without covalent bonding.

2. The nanoparticle according to claim 1, wherein the Gd³⁺ -bearing chemical species is a Gd³⁺ salt or a Gd³⁺ complex, preferably GdCl₃ or Gd-DTPA.

3. The nanoparticle according to claim 1 or 2, wherein albumin is cross-linked with glutaraldehyde.

4. The nanoparticle according to any one of claims 1 to 3, for use as a contrast agent for magnetic resonance imaging (MRI).

5. The nanoparticle for use according to claim 4, in the magnetic resonance imaging (MRI) of tumours.

6. A method of synthesis of cross-linked albumin nanoparticles encapsulating a Gd³⁺-bearing chemical species, comprising the steps of:
- desolvating an aqueous solution comprising albumin and a Gd³⁺-bearing chemical species by the addition to said aqueous solution of a desolvating agent, in order to obtain albumin nanoparticles encapsulating a plurality of molecules of the Gd³⁺ -bearing chemical species; and
- stabilizing the nanoparticles obtained in the previous step by cross-linking of albumin by using a cross-linking agent,
**characterized in that** the concentration of albumin in the desolvation reaction volume is comprised between 0.1 and 0.5% (w/v), that the molar concentration ratio between albumin and the Gd³⁺-bearing chemical species in the desolvation reaction volume is comprised between 1:0.5 and 1:2, and that the desolvating agent is acetone.

7. The method of synthesis according to claim 6, wherein the Gd³⁺ -bearing chemical species is a Gd³⁺ salt or a Gd³⁺ complex, preferably GdCl₃ or Gd-DTPA.

8. The method of synthesis according to claim 6 or 7, wherein in the desolvation step the volume ratio between acetone and water is comprised between 1.25 and 4.

9. The method of synthesis according to any one of claims 6 to 8, wherein the cross-linking is achieved by using a glutaraldehyde aqueous solution at a concentration of between 2 and 8% (v/v).

10. The method according to any one of claims 6 to 9, further comprising a step of purification of the nanoparticles by centrifugation.

## Patentansprüche

1. Quervernetztes Albumin-Nanopartikel, das mit einer Mehrzahl an Molekülen einer Gd³⁺-tragenden chemischen Spezies beladen ist, **dadurch gekennzeichnet, dass** die Größe des Nanopartikels zwischen 50 nm und 80 nm liegt, und dass die Moleküle der Gd³⁺-tragenden chemischen Spezies durch Albumin ohne kovalente Bindung eingekapselt sind.

2. Nanopartikel nach Anspruch 1, wobei die Gd³⁺-tragende chemische Spezies ein Gd³⁺-Salz oder ein Gd³⁺-Komplex, bevorzugt GdCl₃ oder Gd-DTPA, ist.

3. Nanopartikel nach Anspruch 1 oder 2, wobei Albumin mit Glutaraldehyd quervernetzt ist.

4. Nanopartikel nach einem der Ansprüche 1 bis 3, zur Verwendung als ein Kontrastmittel für die Magnetresonanztomographie (MRT).

5. Nanopartikel zur Verwendung nach Anspruch 4, in der Magnetresonanztomographie (MRT) von Tumoren.

6. Verfahren der Synthese von quervernetzten Albumin-Nanopartikeln, die eine Gd³⁺-tragende chemische Spezies einkapseln, umfassend die Schritte:
- Desolvatisieren einer wässrigen Lösung, die Albumin und eine Gd³⁺-tragende chemische Spezies umfasst, durch die Zugabe zu dieser wässrigen Lösung von einem Desolvatisierungsmittel, um Albumin-Nanopartikel zu erhalten, die eine Mehrzahl von Molekülen der Gd³⁺-tragenden chemischen Spezies einkapseln; und
- Stabilisieren der im vorherigen Schritt erhaltenen Nanopartikel durch Quervernetzung von Albumin unter Verwendung eines Quervernetzungsmittels,
**dadurch gekennzeichnet, dass** die Konzentration von Albumin im Desolvatisierungsreaktionsvolumen zwischen 0,1 und 0,5 % (w / v) liegt, dass das molare Konzentrationsverhältnis zwischen Albumin und der Gd³⁺-tragenden chemischen Spezies im Desolvatisierungsreaktionsvolumen zwischen 1 : 0,5 und 1 : 2 liegt, und dass das Desolvatisierungsmittel Aceton ist.

7. Verfahren der Synthese nach Anspruch 6, wobei die Gd³⁺-tragende chemische Spezies ein Gd³⁺-Salz oder ein Gd³⁺-Komplex, bevorzugt GdCl₃ oder Gd-DTPA, ist.

8. Verfahren der Synthese nach Anspruch 6 oder 7, wobei in dem Desolvatisierungsschritt das Volumenverhältnis zwischen Aceton und Wasser zwischen 1,25 und 4 liegt.

9. Verfahren der Synthese nach einem der Ansprüche 6 bis 8, wobei die Quervernetzung unter Verwendung einer wässrigen Glutaraldehydlösung bei einer Konzentration zwischen 2 und 8% (v / v) erreicht wird.

10. Verfahren nach einem der Ansprüche 6 bis 9, ferner umfassend einen Schritt der Reinigung der Nanopartikel durch Zentrifugieren.

## Revendications

1. Nanoparticule d'albumine réticulée chargée avec une pluralité de molécules d'une espèce chimique porteuse de Gd³⁺, **caractérisée en ce que** la taille de la nanoparticule est comprise entre 50 nm et 80 nm et **en ce que** les molécules de l'espèce chimique porteuse de Gd³⁺ sont encapsulées par de l'albumine sans liaison covalente.

2. Nanoparticule selon la revendication 1, dans laquelle l'espèce chimique porteuse de Gd³⁺ est un sel de Gd³⁺ ou un complexe de Gd³⁺, de préférence GdCl₃ ou Gd-DTPA.

3. Nanoparticule selon la revendication 1 ou 2, dans laquelle l'albumine est réticulée avec du glutaraldéhyde.

4. Nanoparticule selon l'une quelconque des revendications 1 à 3, pour une utilisation comme agent de contraste pour l'imagerie par résonance magnétique (IRM).

5. Nanoparticule pour une utilisation selon la revendication 4, dans l'imagerie par résonance magnétique (IRM) de tumeurs.

6. Procédé de synthèse de nanoparticules d'albumine réticulée encapsulant une espèce chimique porteuse de Gd³⁺, comprenant les étapes suivantes :
- désolvater une solution aqueuse comprenant de l'albumine et une espèce chimique porteuse de Gd³⁺ par l'ajout à ladite solution aqueuse d'un agent de désolvatation, afin d'obtenir des nanoparticules d'albumine encapsulant une pluralité de molécules de l'espèce chimique porteuse de Gd³⁺ ; et
- stabiliser les nanoparticules obtenues dans l'étape précédente par la réticulation de l'albumine en utilisant un agent de réticulation,
**caractérisé en ce que** la concentration en albumine dans le volume réactionnel de désolvatation est comprise entre 0,1 et 0,5 % (en poids/volume), **en ce que** le rapport de concentrations molaires entre l'albumine et l'espèce chimique porteuse de Gd³⁺ dans le volume réactionnel de désolvatation est compris entre 1 : 0,5 et 1 : 2, et **en ce que** l'agent de désolvatation est l'acétone.

7. Procédé de synthèse selon la revendication 6, dans lequel l'espèce chimique porteuse de Gd³⁺ est un sel de Gd³⁺ ou un complexe de Gd³⁺, de préférence GdCl₃ ou Gd-DTPA.

8. Procédé de synthèse selon la revendication 6 ou 7, dans lequel dans l'étape de désolvatation, le rapport volumique entre l'acétone et l'eau est compris entre 1,25 et 4.

9. Procédé de synthèse selon l'une quelconque des revendications 6 à 8, dans lequel la réticulation est réalisée en utilisant une solution aqueuse de glutaraldéhyde à une concentration située entre 2 et 8 % (en volume/volume).

10. Procédé selon l'une quelconque des revendications 6 à 9, comprenant en outre une étape de purification des nanoparticules par centrifugation.
